(19) Europäisches Patentamt
European Patent Office
Office européen des brevets

(11) EP 3 275 869 A1

(12) # EUROPEAN PATENT APPLICATION

published in accordance with Art. 153(4) EPC

(43) Date of publication:
**31.01.2018 Bulletin 2018/05**

(21) Application number: **16772723.9**

(22) Date of filing: **28.03.2016**

(51) Int Cl.:
***C07D 233/61*** (2006.01) ***C07C 311/48*** (2006.01)
***C08K 5/3445*** (2006.01) ***H01L 23/36*** (2006.01)
***H01L 23/373*** (2006.01)

(86) International application number:
**PCT/JP2016/059871**

(87) International publication number:
**WO 2016/158842 (06.10.2016 Gazette 2016/40)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **27.03.2015 JP 2015067659**

(71) Applicant: **Kaneka Corporation**
**Osaka-shi, Osaka 530-8288 (JP)**

(72) Inventors:
- **MIYAMOTO, Shinpei**
  **Settsu-shi**
  **Osaka 566-0072 (JP)**
- **NISHIURA, Koichi**
  **Settsu-shi**
  **Osaka 566-0072 (JP)**
- **ONISHI, Yoshifumi**
  **Settsu-shi**
  **Osaka 566-0072 (JP)**

(74) Representative: **Vossius & Partner**
**Patentanwälte Rechtsanwälte mbB**
**Siebertstrasse 3**
**81675 München (DE)**

(54) **HEAT-RESISTANT ALIPHATIC-CHAIN SATURATED HYDROCARBON HAVING ION PAIRS AT BOTH ENDS, AND COMPOSITION USING SAME**

(57) The present invention provides a heat-resistant aliphatic-chain saturated hydrocarbon having ion pairs at both ends and thermally conductive composition or the like containing the heat-resistant aliphatic-chain saturated hydrocarbon. In the present invention, ion pairs are introduced at both ends of an aliphatic-chain saturated hydrocarbon. Thereby, it is possible to provide an organic compound having both high heat resistance and softness that does not volatilize or thermally decompose even in a high-temperature environment.

EP 3 275 869 A1

## Description

[Technical Field]

The present invention relates to a heat-resistant organic compound and a composition using the heat-resistant organic compound.

[Technical Background]

An aliphatic-chain saturated hydrocarbon generally has a linear or branched structure or has a both linear and branched structure. Physical properties such as a melting point, a decomposition temperature and viscosity of an aliphatic-chain saturated hydrocarbon can be significant influenced by a structure and a molecular weight of the aliphatic-chain saturated hydrocarbon, and the aliphatic-chain saturated hydrocarbon can exhibit properties from liquid-like properties (liquid paraffin) to solid-like properties (paraffin wax and polyethylene filler).

Among these, the paraffin wax is a soft solid at a normal temperature and a main component of the paraffin wax is a mixture of normal paraffins having 20 or more carbon atoms. The paraffin wax is used not only in candles and crayons, but also in a very wide range of applications from chemicals to pharmaceuticals. However, at a high temperature, in addition to volatilization, molecular weight reduction due to thermal decomposition progresses and a weight of the paraffin wax significantly decreases, and thus the paraffin wax is not suitable for use at a high temperature as lubricating oil or the like.

On the other hand, existence of an ionic liquid having a melting point of 100 °C or lower and composed only of ions is known. An ionic liquid has a very low vapor pressure, and is a substance that has both features of being non-volatile (for example, Non-Patent Document 1) and being flame-retardant at or below a decomposition temperature. This makes an ionic liquid interesting as an alternative compound in many applications (Non-Patent Document 2). In particular, an ionic liquid is stable against heat and oxidation by forming various strong and weak ionic bonds different from liquid molecules linked by general molecular attractions. Therefore, a lubricating oil using an ionic liquid as a base oil has been proposed (for example, Patent Documents 1 and 2).

However, in recent years, along with progresses in sophistication from Si chips to SiC and GaN chips, IC chips used in electronic devices such as computers generate large amounts of heat, and this causes operation failures due to temperature rising in the chips, and heat stresses cannot be neglected. There are many proposals for a heat dissipation member for suppressing operation temperature rising. However, when a temperature difference is large between when a device or the like is in operation and when the device or the like is not in operation, there is a problem that a coated heat dissipation member cannot follow expansions between a substrate and a heat sink and cannot transmit heat. Therefore, a heat dissipation grease, which is a liquid substance, may be applied between a base substrate, on which an IC chip is mounted, and a heat sink. However, since the heat dissipation grease is usually a liquid, when the heat dissipation grease is repeatedly exposed to a high temperature environment, it is likely that separation between components gradually occurs and an effect as a grease is lost. Therefore, a thermally conductive material mainly composed of paraffin (aliphatic hydrocarbon), which is a solid at normal temperatures and softens at high temperatures to gain followability, has been proposed. However, there is a problem that, as described above, since paraffin is a component, when the thermally conductive material is exposed to a high temperature environment for a long period of time, its functionality is lost due to volatilization or thermal decomposition. In view of such a situation, development of a thermally conductive composition that does not volatilize or thermally decompose in a high temperature environment and follows heat from low temperature to high temperature and has a high thermal conductivity is desired.

[Related Art]

[Patent Documents]

[Patent Document 1] International Publication No. 2005/035702.

[Patent Document 2] Japanese Patent Laid-Open Publication No. 2014-198784.

[Non-Patent Documents]

[Non-Patent Document 1] J. Chem. Soc. Chem. Commun. (1992), pp 965-967.

[Non-Patent Document 2] H. Ohno (Ed.), "Ionic Liquid II-Marvelous Developments and Colorful Near Future" CMC (2006).

[Summary of the Invention]

[Problems to Be Solved by the Invention]

The present invention is accomplished in view of such a situation, and is intended to provide a soft aliphatic-chain saturated hydrocarbon having a low melting point such as paraffin that does not volatilize or thermally decompose even in a high temperature environment, different from a conventional soft organic compound used in heat dissipation grease. Further, the present invention is intended to provide a thermally conductive composition containing the aliphatic-chain saturated hydrocarbon that can change hardness following a heat-generating component and a heat-dissipating component or following a distortion between the two during temperature rising or falling.

[Means for Solving the Problems]

As a result of intensive studies to achieve the above object, the present inventors found that an aliphatic-chain saturated hydrocarbon having ion pairs at ends does not volatilize or thermally decompose and has high heat resistance. Further, the present inventors found that a thermally conductive composition using the aliphatic-chain saturated hydrocarbon has physical properties suitable for use as a thermal interface material (hereinafter, may also be referred to as "TIM").

Specifically, the present inventors found that, by introducing ion pairs at both ends of a simple aliphatic-chain saturated hydrocarbon, the aliphatic-chain saturated hydrocarbon becomes an organic compound that has both a far superior heat resistance and softness as compared to an aliphatic-chain saturated hydrocarbon in which ion pairs are not introduced. Further, the present inventors found that, by mixing one or more kinds of aliphatic-chain saturated hydrocarbons having ion pairs introduced at ends with thermally conductive fillers such as aluminum, hexagonal boron nitride and aluminum oxide, a thermally conductive composition having excellent heat resistance capable of changing hardness following a thermal strain can be obtained, and thus accomplished the present invention.

That is, the present invention includes the following aspects.

(1) A heat-resistant aliphatic-chain saturated hydrocarbon having ion pairs at both ends.

(2) In the aliphatic-chain saturated hydrocarbon described in (1), a hydrocarbon chain portion of the saturated hydrocarbon has 18 or more carbon atoms.

(3) In the aliphatic-chain saturated hydrocarbon described in (1) or (2), an end of the hydrocarbon chain is an onium ion, and an anion exists in a vicinity of the onium ion.

(4) In the aliphatic-chain saturated hydrocarbon described in (3), the onium ion at the end of the hydrocarbon chain has a structure represented by the following general formula (1):

[Chemical Formula 1]

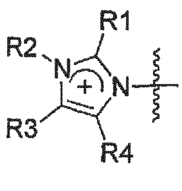

(1)

(where R1 - R4 may be the same or different and each represent a hydrocarbon that has 1 to 12 carbon atoms and may have a hydroxyl group, a halogen atom, a nitro group, a cyano group, a carboxyl group, an alkoxy group, a phenyl group, a phenylene group or a carbonyl group, or a hydrogen atom).

(5) In the aliphatic-chain saturated described in (3) or (4), the anion has a structure represented by the following

general formula (2):

[Chemical Formula 2]

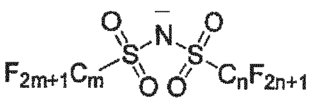

(2)

(where m and n are each an integer from 1 to 8 and may be the same or different).

(6) A thermally conductive composition containing a hydrocarbon represented by the following general formula (3):

[Chemical Formula 3]

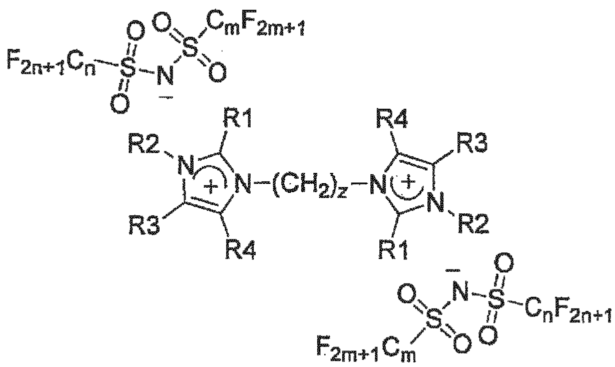

(3)

(where R1 - R4 may be the same or different and each represent a hydrocarbon that has 1 to 12 carbon atoms and may have a hydroxyl group, a halogen atom, a nitro group, a cyano group, a carboxyl group, an alkoxy group, a phenyl group, a phenylene group or a carbonyl group, or a hydrogen atom; m and n are each an integer from 1 to 8, and may be the same or different; and z is an integer of 18 or larger).

(7) A thermal interface material (TIM) containing the thermally conductive composition described in (6).

(8) A thermal interface material (TIM) for a semiconductor module, the thermal interface material (TIM) containing the thermally conductive composition described in (6).

[Effect of the Invention]

The heat-resistant aliphatic-chain saturated hydrocarbon of the present invention is a material having excellent high heat resistance even using an aliphatic-chain saturated hydrocarbon as a main skeleton in which volatilization or thermal decomposition easily occurs. Therefore, the heat-resistant aliphatic-chain saturated hydrocarbon can be suitably used in TIM as a resin component of a thermally conductive composition that does not decompose even in a high temperature environment. In particular, by combining the heat-resistant aliphatic-chain saturated hydrocarbon with thermally conductive fillers such as aluminum, hexagonal boron nitride and aluminum oxide, excellent heat dissipation performance can be achieved.

[Brief Description of the Drawings]

Fig. 1 illustrates thermal weight measurement results of a heat-resistant aliphatic-chain saturated hydrocarbon of Example 1.

Fig. 2 illustrates thermal weight measurement results of a heat-resistant aliphatic-chain saturated hydrocarbon of Example 2.

Fig. 3 illustrates thermal weight measurement results of an aliphatic-chain saturated hydrocarbon of Comparative Example 2.

Fig. 4 illustrates heat resistance test results of the heat-resistant aliphatic-chain saturated hydrocarbon of Example 1 and the aliphatic-chain saturated hydrocarbon of Comparative Example 2.

Fig. 5 illustrates heat resistance test results of a thermally conductive composition of Example 4.

Fig. 6 illustrates heat resistance test results of a thermally conductive composition of Example 5.

Fig. 7 illustrates heat resistance test results of a thermally conductive composition of Example 7.

Fig. 8 illustrates heat resistance test results of a thermally conductive composition of Comparative Example 4.

[Mode for Carrying Out the Invention]

In the following, the present invention is described in detail.

[Heat-Resistant Aliphatic-Chain Saturated Hydrocarbon]

(Synthesis Method)

A synthesis method of an ionic liquid for a thermally conductive composition in an embodiment of the present invention includes the following steps.

Step 1: Under a nitrogen atmosphere, alkanol-1-halide, 4-dimethylaminopyridine, dichloromethane and triethylamine are mixed, and thereafter, p-toluenesulfonyl chloride is added at 0 °C in an ice bath, and the mixture is stirred for 24 hours to allow the components to react while the temperature is gradually raised from 0 °C to room temperature. Triethylamine hydrochloride, which is a by-product, is removed. A saturated solution of sodium chloride is added to the reaction solution, and extraction with dichloromethane is performed. Washing with a saturated solution of sodium chloride is performed. A solvent is distilled off under reduced pressure. A crude product is subjected to silica gel column chromatography to obtain purified alkyl-p-toluenesulfonate-1-halide.

[Chemical Formula 4]

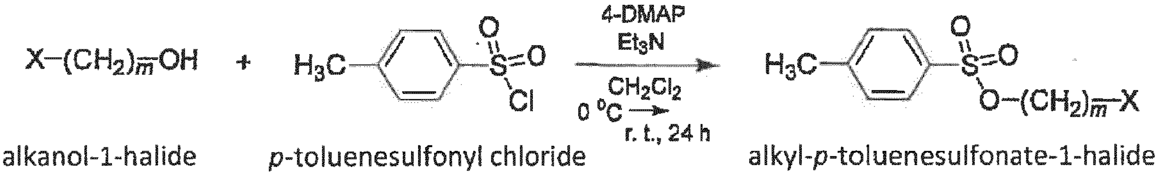


alkanol-1-halide  *p*-toluenesulfonyl chloride  alkyl-*p*-toluenesulfonate-1-halide

where m is an integer of 1 or larger; and X is a chlorine atom or a bromine atom.

In the present invention, it should be understood that (1) it is sufficient to allow alkanol-1-halide and p-toluenesulfonyl chloride to completely react with each other, and the reaction time is not limited to 24 hours; (2) it is sufficient for the reaction to be performed in an inert gas atmosphere, and the inert gas atmosphere is not limited to an atmosphere of a nitrogen gas or an argon gas; and (3) the crude product is not limited to being purified using silica gel column chromatography, and it is also possible that alkyl-p-toluenesulfonate-1-halide is purified using other methods in the art.

Step 2: Under a nitrogen atmosphere, the alkyl-p-toluenesulfonate-1-halide synthesized in Step 1, copper (II) chloride, 1-phenylpropane and dehydrated tetrahydrofuran are mixed and thereafter, a pentamethylene bis(magnesium bromide)-tetrahydrofuran solution is gradually added at 0 °C in an ice bath, and the mixture is stirred for 24 hours to allow the components to react while the temperature is gradually raised from 0 °C to room temperature. After completion of the reaction, a 5% hydrochloric acid is added at 0 °C in an ice bath and the temperature is raised to room temperature. A saturated aqueous ammonium chloride solution is added and the mixture is stirred until an aqueous layer turns blue. A separated organic layer is washed with a 5% hydrochloric acid and a saturated aqueous solution of ammonium chloride, and a solvent is distilled off under reduced pressure to obtain a crude product of alkyl-1,1'-dihalide.

[Chemical Formula 5]

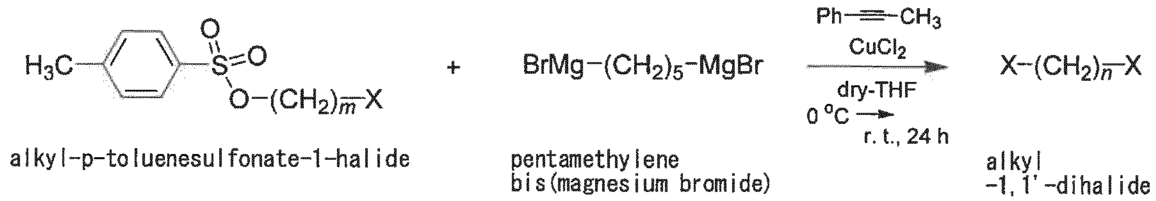


where m is an integer of 1 or larger; n is an integer of 7 or larger; and X is a chlorine atom or a bromine atom.

In the present invention, it should be understood that (1) it is sufficient to allow alkyl-p-toluenesulfonate-1-halide and pentamethylene bis(magnesium bromide) completely react with each other, and the reaction time is not limited to 24 hours; and (2) it is sufficient for the reaction to be performed in an inert gas atmosphere, and the inert gas atmosphere is not limited to an atmosphere of a nitrogen gas or an argon gas. Further, it should be understood that (3) since a p-toluenesulfonyl group in the reaction is an excellent leaving group, even in a situation where a Cl group or a Br group is mixed, a Grignard reaction can highly selectively proceed. Therefore, alkyl-1,1'-dihalide can be obtained without purification. Further, it should also be understood that (4) some alkyldihalides are commercially available, and in this case, a purchased alkyl-1,1'-dihalide may be used.

Step 3: the alkyl-1,1 '-dihalide synthesized in step 2, an onium ion source (amines, phosphines, sulfides or the like) and a reaction solvent are mixed and thereafter the mixture is subjected to heating reflux for 48 hours to allow the components to react with each other. After completion of the reaction, a solvent is distilled off under reduced pressure. The residue is washed with a solvent that dissolves only the onium ion source to obtain alkanediylbis (onium halide). Further, distilled water is added at room temperature to suspend the solution and thereafter a metal salt aqueous solution is added and the mixture is stirred for 24 hours to allow the components to react with each other. After completion of the reaction, a precipitate or an organic material layer is washed with distilled water, and is dried under reduced pressure while being heated to obtain alkanediylbis(onium anion).

[Chemical Formula 6]

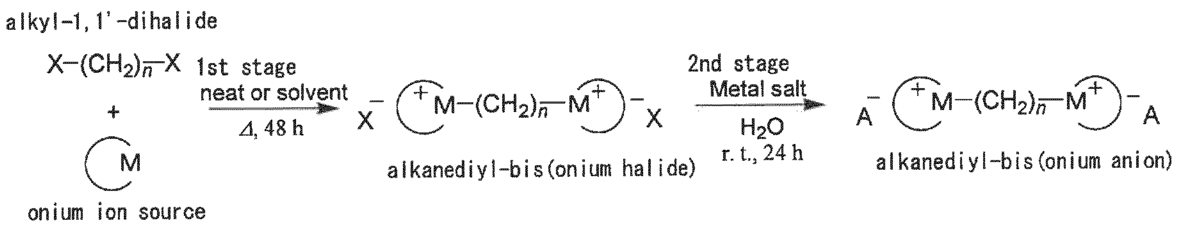


where n is an integer of 4 or larger; M is a nitrogen atom, a phosphorus atom or a sulfur atom; these may be the same or different, and may each be a structure containing the above-mentioned atoms; and X is a chlorine atom or a bromine atom. Further, the metal salt in the reaction of the second stage is a lithium salt, a sodium salt, a magnesium salt, an aluminum salt, or a potassium salt; and A- is an anion other than a chloride ion, a bromide ion and an iodide ion.

In the present invention, it should be understood that (1) the onium ionization reaction of the first stage is a general reaction and is not limited to a structure of the onium ion source; (2) it is sufficient to allow the alkyl-1,1'-dihalide and the onium ion source to completely react with each other, and the reaction time is not limited to 48 hours; and (3) it is sufficient for the reaction to be performed under heating by uniformly transmitting heat and stirring, and a solvent is not necessarily required.

(Molecular Structure)

A heat-resistant aliphatic-chain saturated hydrocarbon of the present invention is characterized by having ion pairs at its both ends. By having ion pairs at its both ends, the heat-resistant aliphatic-chain saturated hydrocarbon of the present invention achieves an effect of having high heat resistance without causing volatilization or thermal decomposition to occur even in a high temperature environment.

The aliphatic-chain saturated hydrocarbon of the present invention has higher heat resistance as compared to an ordinary aliphatic-chain saturated hydrocarbon that does not have ion pairs at both ends. The heat resistance can be evaluated, for example, by tests on [physical properties] or by [heat resistance tests] to be described later.

In the tests on the [physical properties], the heat-resistant aliphatic-chain saturated hydrocarbon of the present invention stably exists up to a decomposition point (350 °C or higher) without volatilization. The decomposition point of the heat-resistant aliphatic-chain saturated hydrocarbon of the present invention is preferably 250 °C or higher, more preferably 300 °C or higher, and even more preferably, 350 °C or higher.

On the other hand, in the [heat resistance tests], when the tests are performed at 170 °C under an air atmosphere, the heat-resistant aliphatic-chain saturated hydrocarbon of the present invention is stably maintained without a significant reduction in weight for a period of preferably 100 hours or more, more preferably 300 hours or more, even more preferably 400 hours or more, and particularly preferably 500 hours or more.

Specifically, the heat-resistant aliphatic-chain saturated hydrocarbon of the present invention has a structure represented by the following general formula (4).

[Chemical Formula 7]

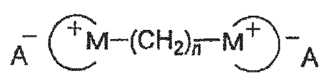

(4)

where n is an integer of 4 or larger, preferably an integer of 17 or larger, more preferably an integer of 18 or larger, and even more preferably an integer of 29 or larger.

Further, in the general formula (4), M is a structure containing at least one of a nitrogen atom, an oxygen atom, a phosphorus atom and a sulfur atom, and a preferred mode thereof will be described later.

Further, in the general formula (4), A- is not particularly limited as long as it is anion other than a chloride ion, a bromide ion and an iodide ion, and a preferred mode thereof will be described later.

In the above general formula (4), the onium ion group (that is, the M+ group in the general formula (4)) is preferably selected from the structures represented by the following general formula (5) or the above-described general formula (1).

[Chemical Formula 8]

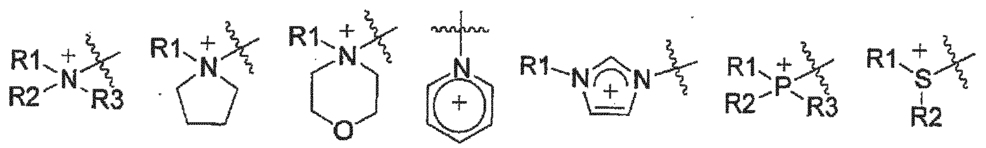

(5)

where R1 - R3 each represent a hydrocarbon that has 1 to 12 carbon atoms and may have a hydroxyl group, a halogen atom, a nitro group, a cyano group, a carboxyl group, an alkoxy group, a phenyl group, a phenylene group or a carbonyl group, or a hydrogen atom. When the hydrocarbons are substituted with the substituent groups, the substituents may be the same or different. Further, from a point of view of heat resistance, R1 - R3 are preferably each a hydrocarbon having 1-10 carbon atoms.

R1 - R3 are particularly preferably each a methyl group having 1 carbon atom.

From the point of view of heat resistance, the onium ion group in the above general formula (4) is preferably imidazolium, pyridinium or phosphonium in the above general formula (5). Further, the onium ion group in the above general formula (4) is preferably imidazolium.

On the other hand, when the above general formula (1) is selected as the onium ion group in the above general formula (4), in the general formula (1), R1 - R4 may be the same or different and each represent a hydrocarbon that has 1 to 12 carbon atoms and may have a hydroxyl group, a halogen atom, a nitro group, a cyano group, a carboxyl group, an alkoxy group, a phenyl group, a phenylene group or a carbonyl group, or a hydrogen atom). When the hydrocarbons are substituted with the substituent groups, the substituents may be the same or different. Further, from a point of view of heat resistance, R1 - R4 are preferably each a hydrocarbon having 1-10 carbon atoms.

Particularly preferably, R1 is a hydrogen atom, R2 is a methyl group, R3 is a hydrogen atom, and R4 is a hydrogen atom.

In the above general formula (4), onium ions represented by M+ may be the same or different at both ends of the aliphatic-chain saturated hydrocarbon. Preferably, the onium ions represented by M+ are imidazolium ions at both

ends of the aliphatic-chain saturated hydrocarbon.

In the above general formula (4), the anion represented by A- is preferably selected from anions having a structure represented by the following general formula (2).

[Chemical Formula 9]

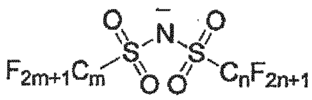

(2)

where m and n are each an integer from 1 to 8 and may be the same or different, and preferably m and n are each 1.

Examples of anions having the structure represented by the above general formula (2) include bis(trifluoromethanesulfonyl) imide, bis(pentafluoroethanesulfonyl) imide, bis(heptafluoropropanesulfonyl) imide, bis(nonafluorobutanesulfonyl) imide, bis(undecafluoropentanesulfonyl) imide, bis(tridecafluorohexanesulfonyl) imide, bis(heptadecafluorooctanesulfonyl) imide, trifluoromethanesulfonyl (pentafluoroethanesulfonyl) imide, trifluoromethanesulfonyl (pentafluoroethanesulfonyl) imide, trifluoromethanesulfonyl (heptafluoropropanesulfonyl) imide, trifluoromethanesulfonyl (nonafluorobutanesulfonyl) imide, trifluoromethanesulfonyl (undecafluoropentanesulfonyl) imide, trifluoromethanesulfonyl (tridecafluorohexanesulfonyl) imide, trifluoromethanesulfonyl (heptadecafluorooctanesulfonyl) imide, pentafluoroethanesulfonyl (heptafluoropropane sulfonyl) imide, pentafluoroethanesulfonyl (nonafluorobutanesulfonyl) imide, pentafluoroethanesulfonyl (tridecafluorohexanesulfonyl) imide, pentafluoroethanesulfonyl (heptadecafluorooctanesulfonyl) imide, heptafluoropropanesulfonyl (nonafluorobutanesulfonyl) imide, nonafluorobutanesulfonyl (tridecafluorohexanesulfonyl) imide, nonafluorobutanesulfonyl (heptadecafluorooctanesulfonyl) imide, and tridecafluorohexanesulfonyl (heptadecafluorooctanesulfonyl) imide.

Among these, from a point of view of heat resistance of the heat-resistant aliphatic-chain saturated hydrocarbon, bis(trifluoromethanesulfonyl) imide, bis(pentafluoroethanesulfonyl) imide, and trifluoromethanesulfonyl (pentafluoroethanesulfonyl) imide are preferred, and bis(trifluoromethanesulfonyl) imide is particularly preferred.

Examples of known anions other than the anions having the structure represented by the above general formula (2) include Cl-, Br-, I-, $RSO_3$-, $RCO_2$-, $NO_3$-, $BF_4$-, $PF_6$-, SCN-, N(CN)-, $C(CN)_3$-, $PF_3(C_2F_5)_3$-, $B(C_2O_4)_2$-, $B(CN)_4$-, $B(C_6H_5)_4$-, and the like. These anions do not have highly conjugated molecular structures, and may have poor thermal stability as compared to the anions having the structure represented by the above general formula (2). Therefore, the anions having the structure represented by the above general formula (2) are more preferably used.

In the general formula (4), anions represented by A- may be the same or different at both ends of the aliphatic-chain saturated hydrocarbon. Preferably, the anions represented by A-are the same at both ends of the aliphatic-chain saturated hydrocarbon.

In the present invention, the heat-resistant aliphatic-chain saturated hydrocarbon having the structure represented by the above general formula (4) preferably can be a hydrocarbon represented by the following general formula (3).

[Chemical Formula 10]

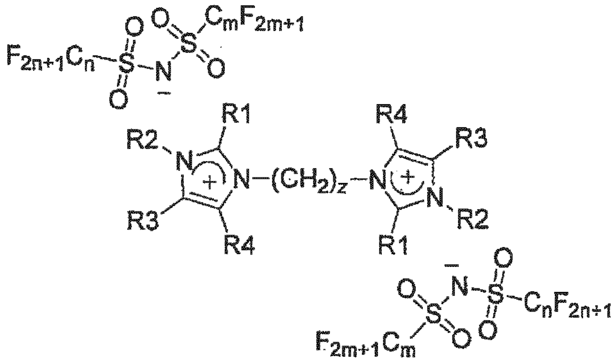

(3)

In the hydrocarbon represented by the above general formula (3), R1 - R4 may be the same or different and each represent a hydrocarbon that has 1 to 12 carbon atoms and may have a hydroxyl group, a halogen atom, a nitro

group, a cyano group, a carboxyl group, an alkoxy group, a phenyl group, a phenylene group or a carbonyl group, or a hydrogen atom). When the hydrocarbons are substituted with the substituent groups, the substituents may be the same or different. Further, from a point of view of heat resistance, R1 - R4 are preferably each a hydrocarbon having 1-10 carbon atoms. Particularly preferably, R1 is a hydrogen atom, R2 is a methyl group, R3 is a hydrogen atom, and R4 is a hydrogen atom.

Further, in the general formula (3), m and n are each an integer from 1 to 8 and may be the same or different, and preferably m and n are each 1.

Further, in the general formula (3), z is an integer of 18 or larger, and preferably an integer of 29 or larger, but is not limited thereto.

[Thermally Conductive Composition]

A thermally conductive composition of an embodiment of the present invention and a preparation method thereof are described in detail.

The thermally conductive composition of the present invention is formed from a resin component and an arbitrary amount of thermally conductive fillers and is characterized in having no reduction in weight in a high temperature environment and having a high thermal conductivity.

The thermally conductive composition of the present invention has higher heat resistance and thermal conductivity as compared to a thermally conductive composition containing an ordinary aliphatic-chain saturated hydrocarbon that does not have ion pairs at both ends. The heat resistance and the thermal conductivity can be evaluated, for example, by [heat resistance tests] or by tests on [thermal conductivity] to be described later.

In the [heat resistance tests], when the tests are performed at 170 °C under an air atmosphere, the thermally conductive composition of the present invention is stably maintained without a significant reduction in weight for a period of preferably 100 hours or more, more preferably 300 hours or more, even more preferably 400 hours or more, and particularly preferably 500 hours or more.

On the other hand, the [thermal conductivity] will be described later.

(Resin Component)

The resin component that forms a part of the thermally conductive composition of the present invention may a mixture obtained by mixing the above-described heat-resistant aliphatic-chain saturated hydrocarbon (preferably, a hydrocarbon represented by the above general formula (3)) and an ionic liquid represented by the following general formula (6) at an arbitrary ratio.

[Chemical Formula 11]

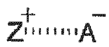 (6)

where Z+ is an onium ion, and A- is an anion.

In the above general formula (6), Z+ is preferably selected from structures represented by the following general formula (7).

[Chemical Formula 12]

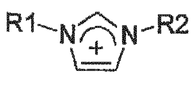 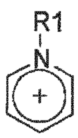 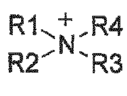 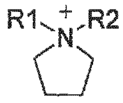 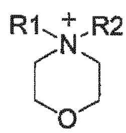 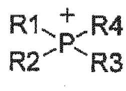 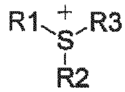 (7)

where R1 - R4 each represent a hydrocarbon that has 1 to 12 carbon atoms and may have a hydroxyl group, a halogen atom, a nitro group, a cyano group, a carboxyl group, an alkoxy group, a phenyl group, a phenylene group or a carbonyl group, or a hydrogen atom. When the hydrocarbons are substituted with the substituent groups, the substituents may be the same or different. Further, from a point of view of heat resistance, R1 - R4 are preferably each a hydrocarbon having 1-10 carbon atoms.

R1 - R4 are particularly preferably each a methyl group having 1 carbon atom.

From the point of view of heat resistance, the onium ion group in the above general formula (6) is preferably imidazolium, pyridinium or phosphonium in the above general formula (7). Further, the onium ion group in the above general formula (6) is preferably imidazolium.

In the above general formula (6), the anion represented by A- is preferably selected from anions having a structure represented by the above general formula (2).

Examples of anions having the structure represented by the above general formula (2) include bis(trifluoromethanesulfonyl) imide, bis(pentafluoroethanesulfonyl) imide, bis(heptafluoropropanesulfonyl) imide, bis(nonafluorobutanesulfonyl) imide, bis(undecafluoropentanesulfonyl) imide, bis(tridecafluorohexanesulfonyl) imide, bis(heptadecafluorooctanesulfonyl) imide, trifluoromethanesulfonyl (pentafluoroethanesulfonyl) imide, trifluoromethanesulfonyl (pentafluoroethanesulfonyl) imide, trifluoromethanesulfonyl (heptafluoropropanesulfonyl) imide, trifluoromethanesulfonyl (nonafluorobutanesulfonyl) imide, trifluoromethanesulfonyl (undecafluoropentanesulfonyl) imide, trifluoromethanesulfonyl (tridecafluorohexanesulfonyl) imide, trifluoromethanesulfonyl (heptadecafluorooctanesulfonyl) imide, pentafluoroethanesulfonyl (heptafluoropropane sulfonyl) imide, pentafluoroethanesulfonyl (nonafluorobutanesulfonyl) imide, pentafluoroethanesulfonyl (tridecafluorohexanesulfonyl) imide, pentafluoroethanesulfonyl (heptadecafluorooctanesulfonyl) imide, heptafluoropropanesulfonyl (nonafluorobutanesulfonyl) imide, nonafluorobutanesulfonyl (tridecafluorohexanesulfonyl) imide, nonafluorobutanesulfonyl (heptadecafluorooctanesulfonyl) imide, and tridecafluorohexanesulfonyl (heptadecafluorooctanesulfonyl) imide.

Among these, from a point of view of heat resistance and compatibility with the heat-resistant aliphatic-chain saturated hydrocarbon, bis(trifluoromethanesulfonyl) imide, bis(pentafluoroethanesulfonyl) imide, and trifluoromethanesulfonyl (pentafluoroethanesulfonyl) imide are preferred, and bis(trifluoromethanesulfonyl) imide is particularly preferred.

Examples of known anions other than the anions having the structure represented by the above general formula (2) include Cl-, Br-, I-, $RSO_3$-, $RCO_2$-, $NO_3$-, $BF_4$-, $PF_6$-, SCN-, N(CN)-, $C(CN)_3$-, $PF_3(C_2F_5)_3$-, $B(C_2O_4)_2$-, $B(CN)_4$-, $B(C_6H_5)_4$-, and the like. These anions do not have highly conjugated molecular structures, and may have poor thermal stability as compared to the anions having the structure represented by the above general formula (2). Therefore, the anions having the structure represented by the above general formula (2) are more preferably used.

(Thermally Conductive Filler)

As a thermally conductive filler used in the present invention, a general commercially available good thermally conductive filler can be used. Among commercially available good thermally conductive fillers, from various points of view, such as thermal conductivity, availability, an insulating property, a filling property, and toxicity, carbon compounds such as graphite (such as spherical graphite) and diamond, metal oxides such as aluminum oxide (such as $\alpha$-alumina (such as rounded or spherical $\alpha$-alumina)),magnesium oxide, beryllium oxide, titanium oxide, zirconium oxide and zinc oxide, metal nitrides such as boron nitride (such as hexagonal boron nitride (such as spherical hexagonal boron nitride)), aluminum nitride and silicon nitride, metal carbides such as boron carbide, aluminum carbide and silicon carbide, fired products of organic polymers such as an acrylonitrile-based polymer fired product, a furan resin fired product, a cresol resin fired product, a polyvinyl chloride fired product, a sugar fired product and a charcoal fired product, composite ferrites of Zn ferrites (such as Mn-Zn based soft ferrites, Ni-Zn based soft ferrites) Fe-Al-Si based ternary alloys (such as spherical Fe-Al-Si based ternary alloys), an iron nickel alloy, metal powder (such as carbonyl iron powder), crystalline silica, and the like can be preferably used.

Further, from a point of view of availability and thermal conductivity, graphite, aluminum oxide, magnesium oxide, boron nitride, aluminum nitride, silicon carbide and crystalline silica are preferable, graphite, $\alpha$-alumina, hexagonal boron nitride, aluminum nitride, Mn-Zn based soft ferrites, Ni-Zn based soft ferrites, Fe-Al-Si based ternary alloys, carbonyl iron and an iron nickel alloy are more preferable, spherical graphite, round or spherical $\alpha$-alumina, spherical hexagonal boron nitride, aluminum nitride, Mn-Zn based soft ferrites, Ni-Zn based soft ferrites, spherical Fe-Al-Si based ternary alloys and carbonyl iron are particularly preferable.

Further, from a point of view of improving dispersibility in a resin, these thermally conductive fillers may be subjected to a surface treatment using a silane coupling agent (such as vinylsilane, epoxysilane, (meth)acrylsilane, isocyanatosilane, chlorosilane, or aminosilane), a titanate coupling agent (such as alkoxy titanate, or aminotitanate), a fatty acid (such as a saturated fatty acid such as a caproic acid, a caprylic acid, a capric acid, a lauric acid, a myristic acid, a palmitic acid, a stearic acid, or behenic acid, or an unsaturated fatty acid such as a sorbic acid, an elaidic acid, an oleic acid, a linoleic acid, a linolenic acid, or an erucic acid) a resin acid (such as an abietic acid, a pimaric acid, a levopimaric acid, a neoapitic acid, a palustric acid, a dehydroabietic acid, an isopimaric acid, a sandaracopimaric acid, a colmic acid, a secodahydroabietic acid, or a dihydroabietic acid), or the like.

From a point of view of increasing the thermal conductivity of the thermally conductive composition of the present invention, an amount of such a thermally conductive filler used is preferably such that a volume ratio (%) (may

be referred to as "vol%" in the present specification) of the thermally conductive filler in the entire composition is 25 vol% or more. When the volume ratio is less than 25 vol%, the thermal conductivity tends to become insufficient. When a higher thermal conductivity is desired, the amount of the thermally conductive filler used is more preferably 40 vol% or more in the entire composition. The thermally conductive composition of the present invention allows a soft sheet to be formed even when the amount of the thermally conductive filler used is extremely high such as 55.0 vol% or more, 65.0 vol% or more, or 72.1 vol% or more, and allows a high thermal conductivity to be achieved.

An upper limited of the amount of the thermally conductive filler used is not particularly limited as long as the thermally conductive composition of the present invention allows a soft sheet to be formed and allows a high thermal conductivity to be achieved.

Here, the volume ratio (%) of the thermally conductive filler is calculated from weight ratios and specific gravities of the resin component and the thermally conductive filler, and is obtained from the following formula. In the following formula (1), the thermally conductive filler is referred to as "filler."

$$\text{Filler volume ratio (\%)} = [(\text{filler weight ratio})/(\text{filler specific gravity})]/[(\text{resin component weight ratio})/(\text{resin component specific gravity}) + (\text{filler weight ratio})/(\text{filler specific gravity})] \times 100$$

Here, the resin component means all components excluding the thermally conductive filler.

Further, as a method of increasing a filling rate of the thermally conductive filler with respect to the resin, it is preferable to use in combination two or more thermally conductive fillers having different particle sizes. In this case, a particle size ratio of a thermally conductive filler having a largest particle size to a thermally conductive filler having a smallest particle size is preferably about 10/1, but is not limited thereto.

Further, not only two or more thermally conductive fillers of the same kind but also two or more thermally conductive fillers of different kinds can be used in combination. Further, various fillers other than thermally conductive fillers may be used as needed to an extent that does not interfere with the effect of the present invention. Various fillers other than thermally conductive fillers are not particularly limited. Examples of fillers other than thermally conductive fillers include reinforcing fillers such as wood flour, pulp, cotton chips, asbestos, glass fiber, carbon fiber, mica, walnut shell powder, rice flour powder, diatomaceous earth, white clay, silica (such as fumed silica, precipitated silica, fused silica, dolomite, silicic anhydride, amorphous spherical silica), and carbon black, fillers of resin powder and the like such as diatomaceous earth, baked clay, clay, talc, titanium oxide, bentonite, organic bentonite, ferric oxide, aluminum fine powder, flint powder, active zinc white, zinc powder, zinc carbonate and shirasu balloon, PVC powder, and PMMA powder, fibrous fillers such as asbestos, glass fiber and glass filaments, carbon fiber, Kevlar fiber, and polyethylene fiber, and the like. Among these fillers, precipitated silica, fumed silica, fused silica, dolomite, carbon black, titanium oxide, talc and the like are preferable. Some of these fillers slightly function as thermally conductive fillers. Further, some of these fillers, such as carbon fibers, various metal powders, various metal oxides, and various organic fibers, can be used as excellent thermally conductive fillers, depending on compositions, synthesis methods, degrees of crystallinity, and crystal structures.

(Additives)

For the thermally conductive composition of the present invention, a composition is used containing at least a heat-resistant aliphatic-chain saturated hydrocarbon, an ionic liquid for dilution and a thermally conductive filler. In addition to these, when necessary, thermal anti-aging agents of the heat-resistant aliphatic-chain saturated hydrocarbon and the ionic liquid for dilution, a plasticizer, a bulking agent, a thixotropic agent, an adhesion imparting agent, a dehydrating agent, a coupling agent, a flame retardant, a filler, a solvent, and the like may be added.

[Thermal Conductivity of Thermally Conductive Composition]

The thermally conductive composition needs to effectively transfer heat to outside. Specifically, the thermal conductivity is 0.9 W/(m·K) or more, preferably 1.5 W/(m·K) or more, and more preferably 2.0 W/(m·K) or more. An upper limit of the thermal conductivity is not particularly limited as long as desired heat conduction in the thermally conductive composition of the present invention can be achieved. By using such a thermally conductive composition, it is possible to efficiently release heat of a heating element, as compared to a case where the heating element is in contact with the air. The thermal conductivity measurement of the thermally conductive composition was performed using a hot disc method thermophysical property measuring device (TPA-501) manufactured by Kyoto Electronics Industry Co., Ltd., at 23 °C by sandwiching a sensor with two square sheet-like samples of 15 mm × 15 mm with a thickness of 3 mm.

[Examples]

In the following, the present invention will be described in more detail with Examples. However, the present invention is not limited by these Examples.

[Synthesis of Heat-Resistant Aliphatic-Chain Saturated Hydrocarbon]

(Example 1)

Step 1: Under a nitrogen atmosphere, dichloromethane (50 mL), 1-bromododecanol (65.5 g, 247 mmol), 4-dimethylaminopyridine (287 mg, 2.35 mmol) and triethylamine (172 mL, 1.23 mol) were added in this order to and were dissolved in a 500 mL three-necked flask equipped with a mechanical stirrer and a thermometer,. The mixed solution was cooled to 0 °C in an ice bath and p-toluenesulfonyl chloride (53.2 g, 279 mmol) was added little by little while the temperature in the system is maintained at 10 °C or lower. After the addition, the mixture was stirred for 24 hours while the temperature was gradually raised from 0 °C to room temperature. After completion of the reaction, triethylamine hydrochloride as a by-product was separated by filtration, a saturated solution of sodium chloride (200 mL) was added to the filtrate, and extraction with dichloromethane (50 mL × 3) was performed. An extracted organic layer was washed with a saturated solution of sodium chloride (100 mL × 3) and was dried over magnesium sulfate. The magnesium sulfate was separated by filtration, and a solvent was distilled off from the filtrate under reduced pressure to obtain a crude product (80.4 g). The crude product was subjected to silica gel column chromatography to obtain 1-bromododecyl-p-toluenesulfonate (75.6 g, 180 mmol, yield: 73%) as a white solid from a toluene fraction.

Step 2: Under a nitrogen atmosphere, dehydrated tetrahydrofuran (52 mL), 1-bromododecyl-p-toluenesulfonate (20.0 g, 55.0 mmol), copper (II) chloride (176 mg, 1.31 mmol) and 1-phenylpropane (609 mg, 5.24 mmol) were added in this order to and were dissolved in a 500 mL three-necked flask equipped with a mechanical stirrer, a thermometer and a dropping funnel with a side tube of 100 mL. The mixed solution was cooled to 0 °C in an ice bath, and pentamethylene bis (magnesium bromide)-tetrahydrofuran solution (55 mL, 27.5 mmol, 0.5 M) was gradually added from the dropping funnel with the 100 mL side tube while the temperature in the system was maintained at 10 °C or lower. After the addition, the mixture was stirred for 24 hours while the temperature was gradually raised from 0 °C to room temperature. After completion of the reaction, cooling to 0 °C was performed in an ice bath, a 5% hydrochloric acid (150 mL) was slowly added and the temperature was raised to room temperature, and then a saturated aqueous ammonium chloride solution (100 mL) was added and the mixture was stirred until an aqueous layer turned blue. An organic layer and an aqueous layer were separated with a separating funnel, and the aqueous layer was extracted with toluene (50 mL × 3). The separated organic layer and the toluene extract were mixed and the mixture was washed with a 5% hydrochloric acid (150 mL) and a saturated aqueous ammonium chloride solution (150 mL × 2) in this order, and was dried over magnesium sulfate. The magnesium sulfate was separated by filtration, and a solvent was distilled off from the filtrate under reduced pressure to obtain 1,29-dibromononacosane (15.7 g as a crude product; theoretical yield: 15.6 g) (which was directly used in the next reaction without purification).

Step 3: The 1,29-dibromononacosane (15.7 g), tetrahydrofuran (10 mL) and 1-methylimidazole (5.02 g, 61.1 mmol) were added in this order to and were heated under reflux at 70 - 80 °C for 42 hours in a 200 mL three-necked flask equipped with a Dimroth condenser. After cooling to room temperature was performed, a solvent was distilled off under reduced pressure, and n-hexane (100 mL) was added and solidification and precipitation were caused to proceed. The precipitate was collected by filtration and was washed with n-hexane, and then was dried under reduced pressure at 60 °C, and 1,1'-(1,29-nonacosanediyl) bis (3-methyl-2H-imidazolium bromide) (19.0 g as a crude product; theoretical yield: 20.3 g) was obtained (which was directly used for the next reaction without purification). Subsequently, 1,1'-(1,29-nonacosanediyl) bis (3-methyl-2H-imidazolium bromide) (10 g) and distilled water (150 mL) were added to and suspended in a 300 mL eggplant type flask. Lithium bis(trifluoromethanesulfonylimide) aqueous solution (4.5 mL, 6.4 M) was slowly added at room temperature and stirred for 24 hours. After completion of the reaction, precipitate was washed with distilled water and dried at 130°C under reduced pressure, and 1,1'-(1,29-nonacosanediyl) bis[3-methyl-2H-imidazolium bis(trifluoromethanesulfonylimide)] (10.6 g; theoretical yield: 15.5 g; specific gravity: 1.26) was obtained.

(Example 2)

Step 1: 1-chlorohexyl-p-toluenesulfonate (53.7 g, 184 mmol, yield: 99%) was obtained by operating in the same manner except that, in the step 1 of the synthesis of the heat-resistant aliphatic-chain saturated hydrocarbon of Example 1, dichloromethane (38 mL), 1-chlorohexanol (25.3 g, 185 mmol), 4-dimethylaminopyridine (215 mg, 1.76 mmol), triethylamine (129 mL, 0.92 mol) and p-toluenesulfonyl chloride (39.8 g, 209 mmol) were used.

Step 2: 1,17-dichloroheptadecane (14.8 g as a crude product; theoretical yield: 15.5 g) was obtained by operating in the same manner except that, in the step 2 of the synthesis of the heat-resistant aliphatic-chain saturated hydrocarbon

of Example 1, dehydrated tetrahydrofuran (100 mL), 1-chlorohexasil-p-toluenesulfonate (30.5 g, 105 mmol), copper (II) chloride (336 mg, 2.50 mmol), a 1-phenylpropane (1.16 g, 10.0 mmol), pentamethylene bis (magnesium bromide)-tetrahydrofuran solution (100 mL, 50.0 mmol), a 5% hydrochloric acid (100 mL) and a saturated aqueous ammonium chloride solution (100 mL) used for quenching, toluene (30 mL × 3) used for extraction, a 5% hydrochloric acid (150 mL) and a saturated aqueous ammonium chloride solution (100 mL × 2) were used.

Step 3: 1,1'-(1,17-heptadecanediyl) bis (3-methyl-2H-imidazolium chloride) (18.5 g as a crude product; theoretical yield: 18.4 g) was obtained by operating in the same manner except that, in the step 3 of the synthesis of the heat-resistant aliphatic-chain saturated hydrocarbon of Example 1, 1,17-dichloroheptadecane (12.0 g), tetrahydrofuran (7 mL), 1-methylimidazole (7.01 g, 85.3 mmol), and n-hexane (130 mL) (for precipitation), were used. Subsequently, 1,1'-(1,17-heptadecanediyl) bis[3-methyl-2H-imidazolium bis(trifluoromethanesulfonylimide)] (23.9 g; theoretical yield: 30.5 g) was obtained by operating in the same manner except that 1,1'-(1,17-heptadecanediyl) bis (3-methyl-2H-imidazolium chloride) (15.0 g), distilled water (30 mL), and a lithium bis(trifluoromethanesulfonylimide) aqueous solution (10 mL, 6.7 M) were used.

(Physical Properties)

Regarding physical properties of the heat-resistant aliphatic-chain saturated hydrocarbons obtained in Examples 1 and 2, results compared with octadecane (Comparative Example 1) and nonacosane (Comparative Example 2), which are aliphatic-chain saturated hydrocarbons, are shown in Table 1, and thermal weight measurement results of Examples 1 and 2 and Comparative Example 2 are shown in Figs. 1 - 3.

[Table 1]

| | Example 1 (Alkyl chain; C29) | Example 2 (Alkyl chain; C17) | Comparative Example 1 Octadecane ($C_{18}H_{38}$) | Comparative Example 2 Nonacosane ($C_{29}H_{60}$) |
|---|---|---|---|---|
| Melting Point | 48 - 50°C (transparent point) | < room temperature | 27 - 29°C (freezing point) | 63 - 66°C |
| TGA (under air atmosphere) | 380°C decomposition point | 373 °C decomposition point | 172°C volatilization point | 244°C volatilization point |
| Property | soft solid (wax) | viscous liquid | crystalline solid | crystalline solid |

The aliphatic-chain saturated hydrocarbons of Comparative Examples 1 and 2 are a crystalline solids and volatilize or decompose at around 170 - 250 °C. On the other hand, the heat-resistant aliphatic-chain saturated hydrocarbon obtained in Example 1 stably exists up to a decomposition point (350 °C or higher) without volatilization despite having the same number of carbon chains as the nonacosane of Comparative Example 2. Further, the introduction of ion pairs into both ends of an aliphatic-chain saturated hydrocarbon allows the aliphatic-chain saturated hydrocarbon that has 18 or more carbon chains and exhibits solid properties to be liquefied or softened, and such an aliphatic-chain saturated hydrocarbon can be suitably used as a component of a thermally conductive composition for a TIM.

(Heat Resistance Tests)

The heat resistance of the heat-resistant aliphatic-chain saturated hydrocarbon obtained in Example 1 was compared with Comparative Example 2 (nonacosane having the same number of carbon chains) in a temperature range (at 170 °C under air atmosphere) where an aliphatic-chain saturated hydrocarbon can easily volatilize or thermally decompose.

From the evaluation results of Fig. 4 that illustrates the heat resistances of the resin components alone, for Comparative Example 2 (aliphatic-chain saturated hydrocarbon), a large weight reduction is observed after 72 hours. On the other hand, for Example 1 (heat-resistant aliphatic-chain saturated hydrocarbon), a large weight reduction is not observed even after 500 hours. Therefore, the heat-resistant aliphatic-chain saturated hydrocarbon of Example 1 does not deteriorate even in long-term use and thus is suitable for use as a component of a thermally conductive composition.

[Manufacture of Thermally Conductive Composition]

Thermally conductive compositions were obtained using material components illustrated below (Examples 3 - 7). Compositions illustrated in the following Comparative Examples are each a composition obtained by kneading an aliphatic-chain saturated hydrocarbon that does not have ion pairs at both ends (Comparative Examples 3-5).
Component (a): (a-1) 1,1'-(1,29-nonacosanediyl) bis[3-methyl-2H-imidazolium bis(trifluoromethanesulfonylimide)]; (a-2) 1,1'-(1,17-heptadecanediyl) bis[3-methyl-2H-imidazolium bis(trifluoromethanesulfonylimide)]; (a-3) octadecane; (a-4) nonacosane.
Component (b): Hexagonal boron nitride (average particle size: 30 μm).
Component (c): Aluminum oxide (average particle size: 0.6 μm).
Component (d): (d-1) aluminum (average particle size: 2 μm); (d-2) aluminum (average particle size: 14 μm).

(Example 3)

In a 200 mL plastic cup made of polypropylene, 1,1'-(1,29-nonacosanediyl) bis[3-methyl-2H-imidazolium bis(trifluoromethanesulfonylimide)] (378 parts by weight), which is the above heat-resistant aliphatic hydrocarbon, was prepared. The hexagonal boron nitride (182 parts by weight), the aluminum oxide (218 parts by weight), the 2 μm aluminum (324 parts by weight) and the 14 μm aluminum (300 parts by weight) were added and thereafter the mixture was kneaded at 100 °C on a hot plate. After cooling, the kneaded product was compressed using a 3 mm stainless steel spacer and a hot press machine (100 °C, 2 - 3 MPa), and a sheet of a thermally conductive composition having a thickness of 3 mm (filler content: 55.0 vol%) was obtained.

(Example 4)

A sheet of a thermally conductive composition (filler content: 65.0 vol%) was obtained by operating in the same manner except that, in the manufacture of the thermally conductive composition of Example 3, the 1,1'-(1,29-nonacosanediyl) bis[3-methyl-2H-imidazolium bis(trifluoromethanesulfonyl imide)] (240 parts by weight), the hexagonal boron nitride (164 parts by weight) and the aluminum oxide (197 parts by weight) were used.

(Example 5)

A sheet of a thermally conductive composition (filler content: 72.1 vol%) was obtained by operating in the same manner except that, in the manufacture of the thermally conductive composition of Example 3, the 1,1'-(1,29-nonacosanediyl) bis[3-methyl-2H-imidazolium bis (trifluoromethanesulfonyl imide)] (240 parts by weight), the hexagonal boron nitride (352 parts by weight) and the aluminum oxide (422 parts by weight) were used.

(Example 6)

A sheet of a thermally conductive composition (filler content: 55.0 vol%) was obtained by operating in the same manner except that, in the manufacture of the thermally conductive composition of Example 3, the 1,1'-(1,17-heptadecanediyl) bis[3-methyl-2H-imidazolium bis (trifluoromethanesulfonylimide)] (378 parts by weight), the hexagonal boron nitride (182 parts by weight) and the aluminum oxide (218 parts by weight) were used.

(Example 7)

A sheet of a thermally conductive composition (filler content: 65.0 vol%) was obtained by operating in the same manner except that, in the manufacture of the thermally conductive composition of Example 3, the 1,1'-(1,17-heptadecanediyl) bis[3-methyl-2H-imidazolium bis (trifluoromethanesulfonylimide)] (240 parts by weight), the hexagonal boron nitride (164 parts by weight) and the aluminum oxide (197 parts by weight) were used.

(Comparative Example 3)

In a 200 mL plastic cup made of polypropylene, octadecane of an aliphatic-chain saturated hydrocarbon (153 parts by weight) was prepared. The hexagonal boron nitride (182 parts by weight), the aluminum oxide (218 parts by weight), the 2 μm aluminum (324 parts by weight) and the 14 μm aluminum (300 parts by weight) were added and thereafter the mixture was kneaded at 100 °C on a hot plate. After cooling, a powdered thermally conductive composition (filler content: 65.1 vol%) was obtained.

(Comparative Example 4)

Operation was performed in the same manner except that, in the manufacture of thermally conductive composition in Comparative Example 3, the nonacosane (241 parts by weight), the hexagonal boron nitride (182 parts by weight) and the aluminum oxide (218 parts by weight) were used. After cooling, the kneaded product was compressed using a 3 mm stainless steel spacer and a hot press machine (100 °C, 2 - 3 MPa), and a sheet of a thermally conductive composition having a thickness of 3 mm (filler content: 54.9 vol%) was obtained.

(Comparative Example 5)

A powdered thermally conductive composition (filler content: 64.3 vol%) was obtained by operating in the same manner except that, in the manufacture of the thermally conductive composition of Comparative Example 3, the the nonacosane (183 parts by weight), the hexagonal boron nitride (164 parts by weight) and the aluminum oxide (197 parts by weight) were used.

Kneading blending ratios and shapes of the thermally conductive compositions obtained in Examples and Comparative Examples are illustrated in Table 2.

[Table 2]

| Thermally conductive composition | Example | | | | | Comparative Example | | |
|---|---|---|---|---|---|---|---|---|
| | 3 | 4 | 5 | 6 | 7 | 3 | 4 | 5 |
| 1,1'-(1,29-nonacosanediyl) bis [3-methyl-2H-imidazolium bis(trifluoromethanesulfonylimide)] | 378 parts by weight | 240 parts by weight | 240 parts by weight | | | | | |
| 1,1'-(1,17-heptadecanediyl) bis[3-methyl-2H-imidazolium bis(trifluoromethanesulfonylimide)] | | | | 378 parts by weight | 240 parts by weight | | | |
| Octadecane | | | | | | 153 parts by weight | | |
| Nonacosane | | | | | | | 241 parts by weight | 183 parts by weight |
| Hexagonal boron nitride | 182 parts by weight | 164 parts by weight | 352 parts by weight | 182 parts by weight | 164 parts by weight | 182 parts by weight | 182 parts by weight | 164 parts by weight |
| Aluminum oxide | 218 parts by weight | 197 parts by weight | 422 parts by weight | 218 parts by weight | 197 parts by weight | 218 parts by weight | 218 parts by weight | 197 parts by weight |
| Aluminum (2 μm) | 324 parts by weight | | | | | | | |
| Aluminum (14 μm) | 300 parts by weight | | | | | | | |
| Filler content | 55.0 vol% | 65.0 vol% | 72.1 vol% | 55.0 vol% | 65.0 vol% | 65.1 vol% | 54.9 vol% | 64.3 vol% |
| Shape | sheet | sheet | sheet | sheet | sheet | powder | sheet | powder |

Aliphatic-chain saturated hydrocarbons used in Comparative Examples 3 - 5 are crystalline solids. Therefore, even when kneaded with thermally conductive fillers, compositions of the aliphatic-chain saturated hydrocarbons are in powder forms rather than in bulk forms. As in Comparative Example 4, it is also possible for the composition to be in a bulk form and molded into a sheet by lowering the filler content. However, the sheet of the composition is very hard and brittle and is not suitable for use as a thermally conductive composition for a TIM. On the other hand, in each of the thermally conductive compositions of Examples 3-7, the heat-resistant aliphatic-chain saturated hydrocarbon itself is a soft solid (wax) or liquid. Therefore, even when the composition is kneaded with a large amount of fillers, a clay-like soft sheet can be obtained.

(Thermal Conductivity)

Results of measurements of thermal conductivities of the thermally conductive compositions obtained in Examples 3 - 7 and Comparative Example 4 are shown in Table 3.

[Table 3]

| Thermally conductive composition | Example | | | | | Comparative Example |
|---|---|---|---|---|---|---|
| | 3 | 4 | 5 | 6 | 7 | 4 |
| Filler content | 55.0 vol% | 65.0 vol% | 72.1 vol% | 55.0 vol% | 65.0 vol% | 54.9 vol% |
| Thermal conductivity | 2.4 W/(m·K) | 3.6 W/(m·K) | 6.0 W/(m·K) | 2.3 W/(m·K) | 3.1 W/(m·K) | 2.5 W/(m·K) |

Comparative Example 4 shows a thermal conductivity of about 2.5 W/(m·K). However, the sheet of Comparative Example 4 is very hard and brittle and thus is not suitable for use as a thermally conductive composition. On the other hand, in each of Examples 3-7, even when kneaded with thermally conductive fillers in an amount equal to or larger than that in Comparative Example 4, the thermally conductive composition allows a soft sheet to be formed and allows a high thermal conductivity (6 W/(m·K)) to observed in a filler content region where an aliphatic-chain saturated hydrocarbon cannot be kneaded.

(Heat Resistance Tests)

Results of heat resistance tests (170 °C, 21 days) of the thermally conductive compositions of Examples 3, 5 and 7 and Comparative Example 4 are shown in Figs. 5-8.

From the evaluation results illustrated in Figs. 5 - 8, for each of the thermally conductive compositions of Examples 3, 5 and 7, weigh change in a high temperature environment is substantially not observed and there is no outgassing due to volatilization and thermal decomposition. On the other hand, for Comparative Example 4, since a aliphatic-chain saturated hydrocarbon is used for a resin component, the composition has poor heat resistance, and a significant weight reduction due to volatilization and outgassing in a high temperature environment is observed, and it is clear that the composition is inappropriate as a thermally conductive composition.

In the present invention, the heat-resistant aliphatic-chain saturated hydrocarbon having ion pairs at both ends has excellent heat resistance even in a high temperature environment, and a solid aliphatic-chain saturated hydrocarbon can be liquefied or softened. A thermally conductive composition using the heat-resistant aliphatic-chain saturated hydrocarbon of the present invention has no weight reduction due to volatilization or decomposition and can withstand long-term use, and has a clay-like softness, and thus is suitable for use as a thermally conductive composition component for a TIM.

[Industrial Applicability]

The heat-resistant aliphatic-chain saturated hydrocarbon of the present invention, as a material of a thermally conductive composition, for example, can be used as a thermally conductive composition component for a TIM.

## Claims

1. A heat-resistant aliphatic-chain saturated hydrocarbon having ion pairs at both ends.

**2.** The aliphatic-chain saturated hydrocarbon according to claim 1, wherein a hydrocarbon chain portion of the saturated hydrocarbon has 18 or more carbon atoms.

**3.** The aliphatic-chain saturated hydrocarbon according to any one of claims 1 and 2, wherein
an end of the hydrocarbon chain is an onium ion, and
an anion exists in a vicinity of the onium ion.

**4.** The aliphatic-chain saturated hydrocarbon according to claim 3, wherein the onium ion at the end of the hydrocarbon chain has a structure represented by the following general formula (1):

[Chemical Formula 1]

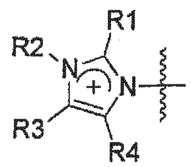

(1)

(where R1 - R4 may be the same or different and each represent a hydrocarbon that has 1 to 12 carbon atoms and may have a hydroxyl group, a halogen atom, a nitro group, a cyano group, a carboxyl group, an alkoxy group, a phenyl group, a phenylene group or a carbonyl group, or a hydrogen atom).

**5.** The aliphatic-chain saturated hydrocarbon according to any one of claims 3 and 4, wherein the anion has a structure represented by the following general formula (2):

[Chemical Formula 2]

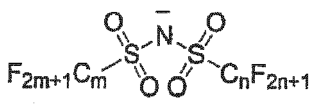

(2)

(where m and n are each an integer from 1 to 8 and may be the same or different).

**6.** A thermally conductive composition containing a hydrocarbon represented by the following general formula (3):

[Chemical Formula 3]

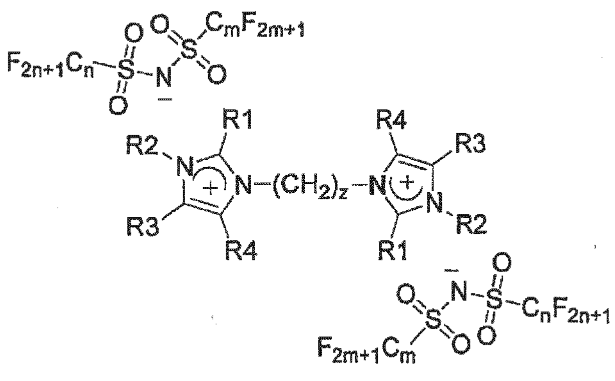

(3)

(where R1 - R4 may be the same or different and each represent a hydrocarbon that has 1 to 12 carbon atoms and may have a hydroxyl group, a halogen atom, a nitro group, a cyano group, a carboxyl group, an alkoxy group, a

phenyl group, a phenylene group or a carbonyl group, or a hydrogen atom; m and n are each an integer from 1 to 8, and may be the same or different; and z is an integer of 18 or larger).

**7.** A thermal interface material (TIM) containing the thermally conductive composition according to claim 6.

**8.** A thermal interface material (TIM) for a semiconductor module, the thermal bonding material (TIM) containing the thermally conductive composition according to claim 6.

FIG.1

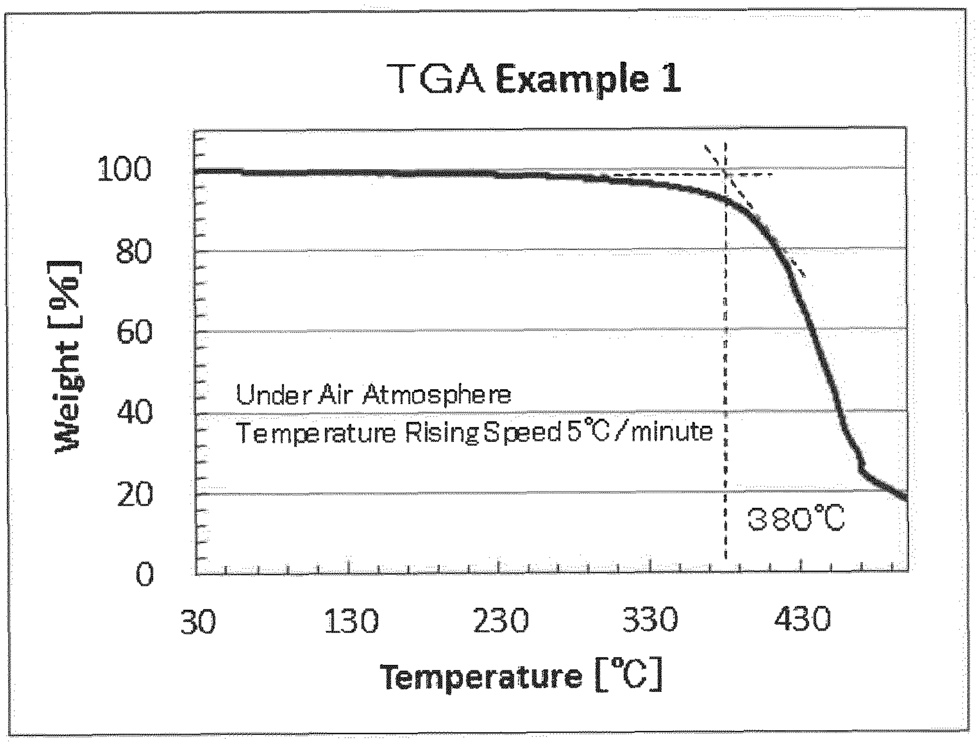
TGA Example 1
Weight [%]
100
80
60
40
20
0
Under Air Atmosphere
Temperature Rising Speed 5°C/minute
380°C
30
130
230
330
430
Temperature [°C]

FIG.2

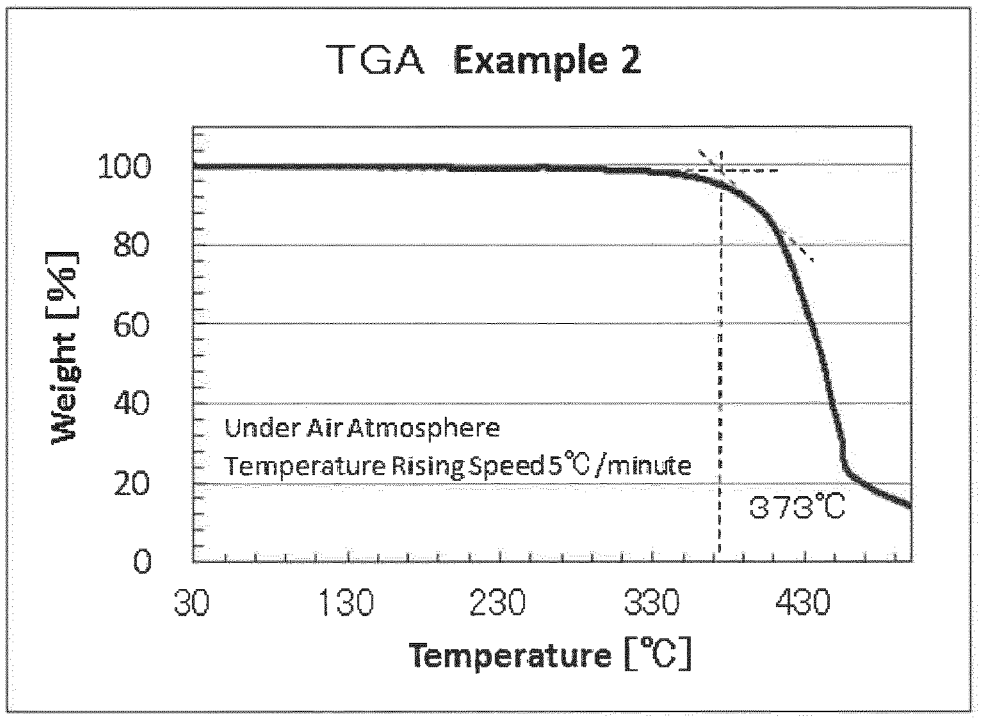
TGA Example 2
Weight [%]
100
80
60
40
20
0
30
130
230
330
430
Temperature [℃]
Under Air Atmosphere
Temperature Rising Speed 5℃/minute
373℃

FIG.3

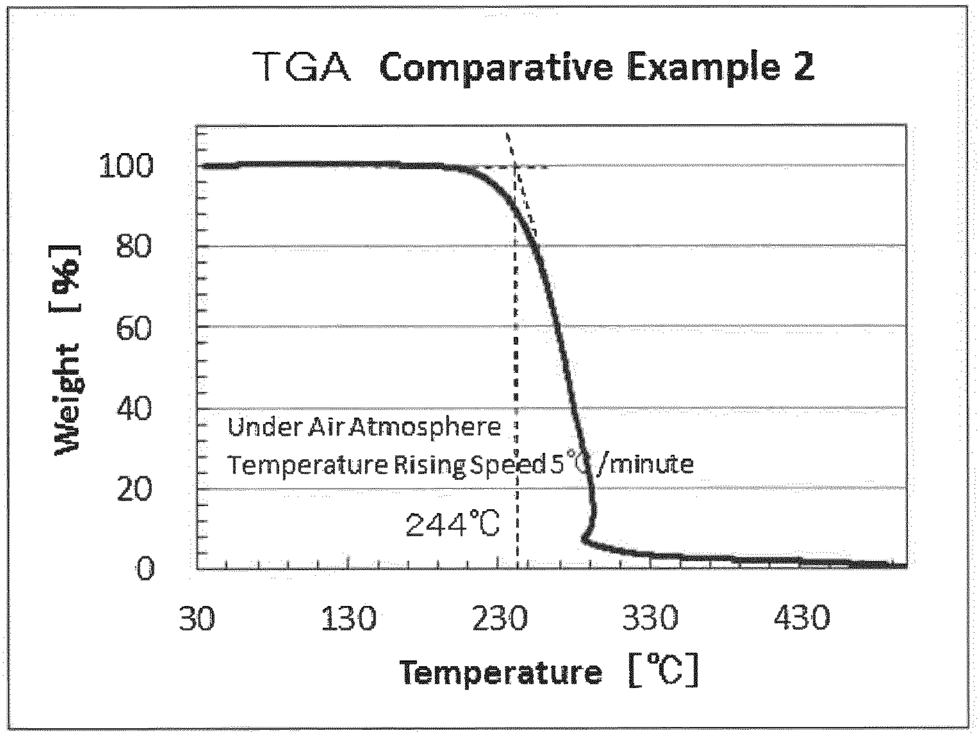
TGA Comparative Example 2
100
80
60
40
20
0
Weight [%]
Under Air Atmosphere
Temperature Rising Speed 5℃/minute
244℃
30
130
230
330
430
Temperature [℃]

FIG.4

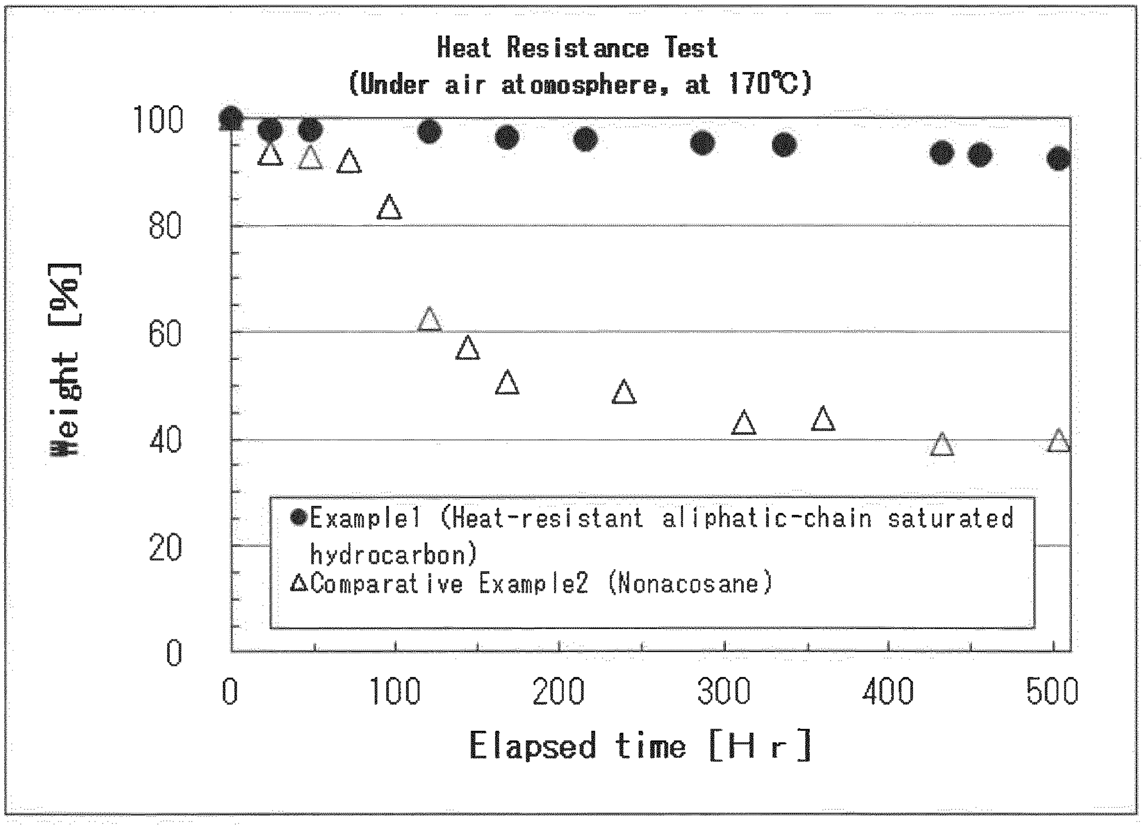
Heat Resistance Test
(Under air atomosphere, at 170℃)
Weight [%]
100
80
60
40
20
0
0
100
200
300
400
500
Elapsed time [Hr]
●Example1 (Heat-resistant aliphatic-chain saturated hydrocarbon)
△Comparative Example2 (Nonacosane)

FIG.5

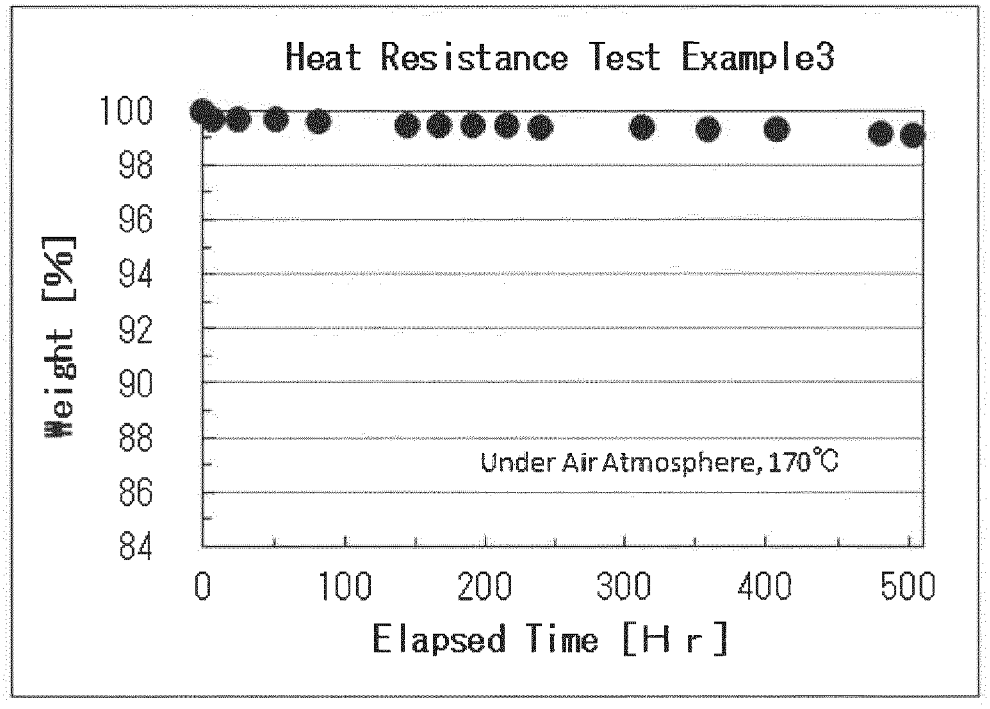
Heat Resistance Test Example3
100
98
96
94
92
90
88
86
84
Weight [%]
Under Air Atmosphere, 170°C
0
100
200
300
400
500
Elapsed Time [Hr]

FIG.6

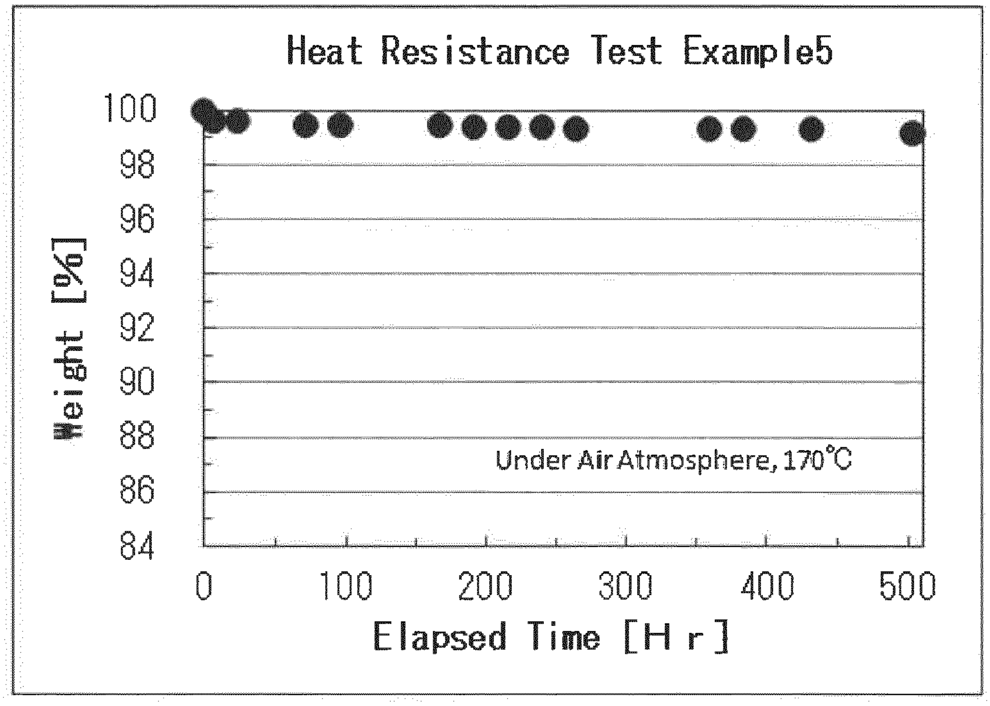
Heat Resistance Test Example5
Weight [%]
100
98
96
94
92
90
88
86
84
0
100
200
300
400
500
Elapsed Time [H r]
Under Air Atmosphere, 170°C

FIG.7

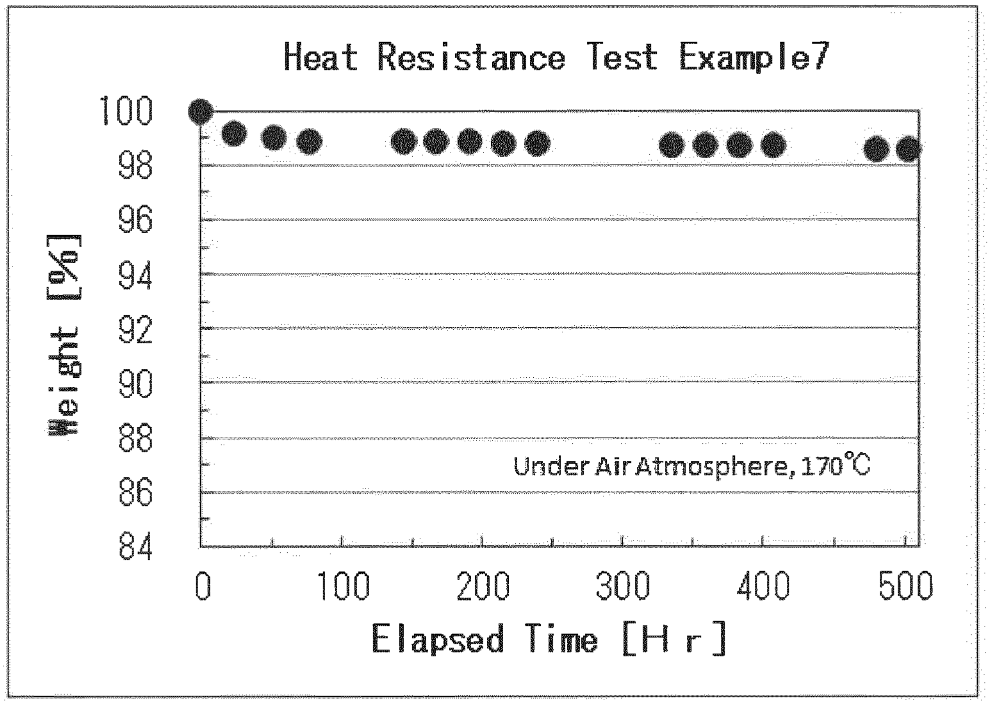
Heat Resistance Test Example7
100
98
96
94
92
90
88
86
84
Weight [%]
Under Air Atmosphere, 170℃
0
100
200
300
400
500
Elapsed Time [H r]

FIG.8

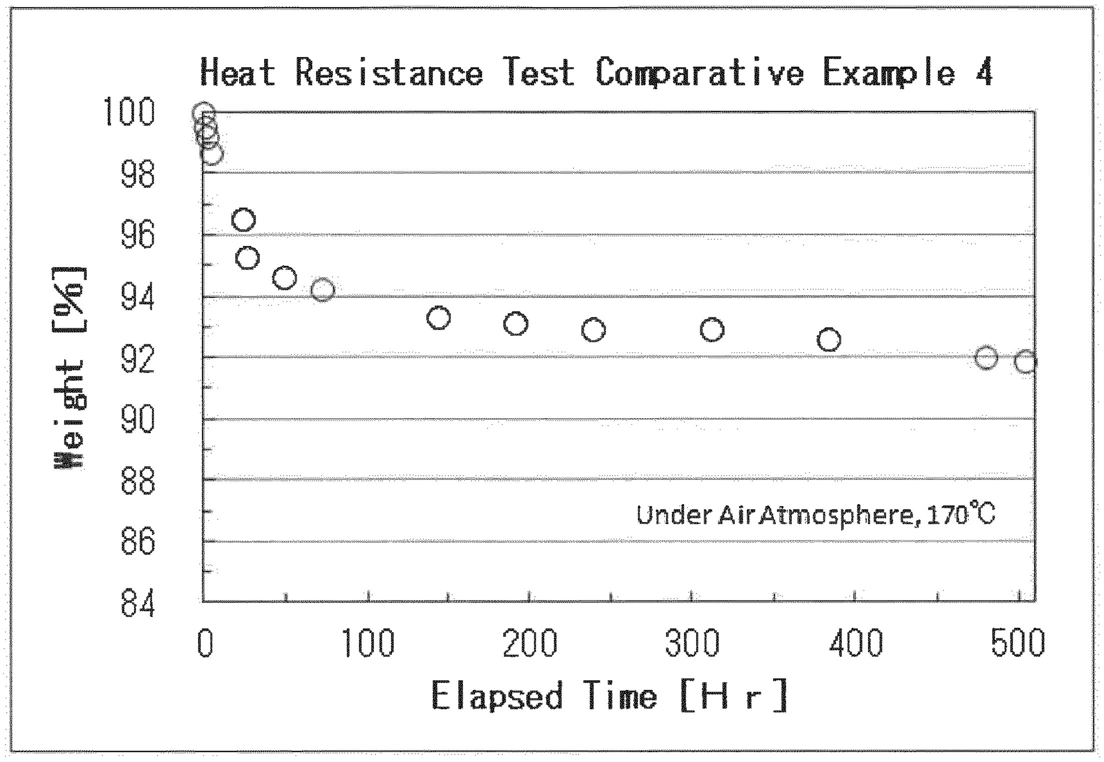
Heat Resistance Test Comparative Example 4
100
98
96
94
92
90
88
86
84
Weight [%]
0
100
200
300
400
500
Elapsed Time [H r]
Under Air Atmosphere, 170℃

## INTERNATIONAL SEARCH REPORT

International application No.
PCT/JP2016/059871

A. CLASSIFICATION OF SUBJECT MATTER
*C07D233/61*(2006.01)i, *C07C311/48*(2006.01)i, *C08K5/3445*(2006.01)i, *H01L23/36*(2006.01)i, *H01L23/373*(2006.01)i

According to International Patent Classification (IPC) or to both national classification and IPC

B. FIELDS SEARCHED

Minimum documentation searched (classification system followed by classification symbols)
C07D233/61, C07C311/48, C08K5/3445, H01L23/36, H01L23/373

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2016 |
| Kokai Jitsuyo Shinan Koho | 1971-2016 | Toroku Jitsuyo Shinan Koho | 1994-2016 |

Electronic data base consulted during the international search (name of data base and, where practicable, search terms used)
CAplus/REGISTRY(STN)

C. DOCUMENTS CONSIDERED TO BE RELEVANT

| Category* | Citation of document, with indication, where appropriate, of the relevant passages | Relevant to claim No. |
|---|---|---|
| X<br>Y | JP 2013-227518 A (Idemitsu Kosan Co., Ltd.), 07 November 2013 (07.11.2013), entire text; particularly, claims; paragraphs [0014], [0025] (Family: none) | 1-5<br>6-8 |
| X<br>Y | JP 2013-227517 A (Idemitsu Kosan Co., Ltd.), 07 November 2013 (07.11.2013), entire text; particularly, claims; paragraphs [0007], [0027] (Family: none) | 1-5<br>6-8 |
| X | GB 745956 B (EDWARD PERCIVAL TAYLOR), 07 March 1956 (07.03.1956), entire text; particularly, claims; examples 10, 11 (Family: none) | 1-3 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 08 June 2016 (08.06.16) | 21 June 2016 (21.06.16) |

| Name and mailing address of the ISA/ | Authorized officer |
|---|---|
| Japan Patent Office<br>3-4-3,Kasumigaseki,Chiyoda-ku,<br>Tokyo 100-8915,Japan | Telephone No. |

Form PCT/ISA/210 (second sheet) (January 2015)

**INTERNATIONAL SEARCH REPORT**

International application No. PCT/JP2016/059871

C (Continuation). DOCUMENTS CONSIDERED TO BE RELEVANT

| Category* | Citation of document, with indication, where appropriate, of the relevant passages | Relevant to claim No. |
|---|---|---|
| X<br>Y | WO 2007/124397 A2 (TRUSTEES OF BOSTON UNIVERSITY), 01 November 2007 (01.11.2007), entire text; particularly, claims; examples 13 to 16; fig. 4<br>& EP 2016056 A1 & US 2009/0176956 A1<br>& US 2013/0204010 A1 & US 2014/0058047 A1 | 1-5<br>6-8 |
| X | WO 2010/025558 A1 (UNIVERSITY HEALTH NETWORK), 11 March 2010 (11.03.2010), entire text; particularly, claims; Scheme 4; table 1<br>& US 2011/0257235 A1 | 1-4 |
| X | WANG,Shuo et al, JOURNAL OF BACTERIOLOGY, 2014, Vol.196, No.17, pp.3122-3133, particularly, TABLE 3, compound starting with QT | 1-4 |
| X | WO 2014/175449 A1 (The University of Tokyo), 30 October 2014 (30.10.2014), entire text; particularly, claims; general formula (1); compounds 1, 2<br>(Family: none) | 1-5 |
| X<br>Y | US 2011/0068306 A1 (Liao Cheng-Chung), 24 March 2011 (24.03.2011), entire text; particularly, claims; formulae (II), (IV); examples 2-1, 2-2<br>& TW 201111349 A | 1-5<br>6-8 |
| X | JP 2010-220490 A (Toyota Central Research and Development Laboratories, Inc.), 07 October 2010 (07.10.2010), entire text; particularly, claims; example 3(8)<br>(Family: none) | 1-5 |
| X<br>Y | JP 2010-518018 A (Sigma-Aldrich Co.), 27 May 2010 (27.05.2010), entire text; particularly, claims; paragraph [0015]; fig. 3; table 10<br>& WO 2006/012513 A2<br>entire text; particularly, claims; paragraph [0024]; table 10; fig. 3<br>& US 2006/0025598 A1 & CA 2574460 A<br>& AU 2005267033 A & EP 1773785 A1<br>& MX 2007000868 A & CN 101061099 A<br>& US 2008/0027231 A1 & JP 2008-507556 A<br>& WO 2008/095064 A2 & EP 2114968 A1<br>& JP 2010-518018 A & US 2012/0211696 A1 | 1-5<br>6-8 |

Form PCT/ISA/210 (continuation of second sheet) (January 2015)

**INTERNATIONAL SEARCH REPORT**

International application No. PCT/JP2016/059871

C (Continuation). DOCUMENTS CONSIDERED TO BE RELEVANT

| Category* | Citation of document, with indication, where appropriate, of the relevant passages | Relevant to claim No. |
|---|---|---|
| X<br>Y | WO 01/04097 A2 (COVALENT ASSOCIATES, INC.), 18 January 2001 (18.01.2001), entire text; particularly, claims; examples 2, 5<br>& GB 16948 D0 & GB 2353997 A<br>& FR 2798383 A & US 6531241 B1 | 1-5<br>6-8 |
| X | JP 2009-135199 A (Fujifilm Corp.), 18 June 2009 (18.06.2009), entire text; particularly, claims; general formula (1); exemplified compounds (1-1) to (1-12), (1-15) to (1-20), (1-23) to (1-24)<br>& TW 200930800 A | 1-5 |
| Y | JP 2007-281048 A (Kaneka Corp.), 25 October 2007 (25.10.2007), entire text<br>(Family: none) | 6-8 |
| A | JP 2010-165868 A (3M Innovative Properties Co.), 29 July 2010 (29.07.2010), entire text<br>& WO 2010/083075 A1 & KR 10-2011-0110796 A<br>& US 2011/0268950 A1 & EP 2387866 A1<br>& CN 102349364 A | 6-8 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2014198784 A


### Non-patent literature cited in the description


- *J. Chem. Soc. Chem. Commun.,* 1992, 965-967